# EUROPÄISCHE PATENTSCHRIFT

(11) **EP 1 389 946 B1**
(45) Veröffentlichungstag und Bekanntmachung des Hinweises auf die Patenterteilung: **31.08.2011**
(21) Anmeldenummer: 02738099.7
(22) Anmeldetag: 23.05.2002
(51) Int. Cl.: A61B 5/00

(54) **POTENTIALFREIE WECHSELSTROMQUELLE**
TRANSFORMER-ISOLATED ALTERNATING CURRENT POWER SUPPLY
SOURCE DE COURANT ALTERNATIF ISOLEE PAR TRANSFORMATEUR

(30) Priorität: 23.05.2001 DE 10125359
(43) Veröffentlichungstag der Anmeldung: 25.02.2004
(60) Teilanmeldung: 11000829.9
(73) Patentinhaber: Osypka Medical GmbH, 12489 Berlin (DE)
(72) Erfinder: GERSING, Eberhard, 37075 Göttingen (DE)
(74) Vertreter: Behrens, Dieter
(86) Internationale Anmeldenummer: PCT/EP2002/005665
(87) Internationale Veröffentlichungsnummer: WO 2002/094090

(56) Entgegenhaltungen:
- US-A- 3 284 724
- US-A- 3 601 126
- US-A- 3 971 365

## Beschreibung

Die Erfindung betrifft eine potentialfreie Wechselstromquelle, wie sie bei der Messung von Impedanzen am menschlichen Körper einsetzbar ist.

Die Impedanz- (oder Admittanz-)Messung an biologischen Geweben, z. B. am menschlichen Körper, ermöglicht Aussagen über deren Zustand, wenn geeignete Frequenzen verwendet werden. Beispielsweise können bei Herzpatienten im Verlauf von und nach Herzoperationen aufgrund der Impedanzmessung für die Diagnose wertvolle Aussagen gewonnen werden. Dasselbe gilt auch bei Organtransplantationen zur Bestimmung der Ischämie-bedingten Schädigung und/oder Wiedererholung des transplantierten Organs.

Für die elektrischen Messungen an inneren Organen des Menschen gelten strenge gesetzliche Sicherheitsvorschriften. Insbesondere muß gewährleistet sein, dass kein Fehlerstrom, der 10 µA überschreitet, über den Menschen abfließt und dass zwischen dem Netz, über das die verwendeten Meßgeräte betrieben werden, und dem menschlichen Meßobjekt eine Isolationsbarriere besteht, die einem Potentialunterschied von 4 kV_{eff} standhält.

Herkömmliche Impedanzmeßgeräte, die u. a. für die Nachrichtentechnik bestimmt sind, erfüllen diese Sicherheitsvorschriften nicht.

Die Impedanzmessung kann vierpolig oder zweipolig erfolgen. Bei einer zweipoligen Anwendung werden an dem Patienten zwei Elektroden angelegt. Dieselben Elektroden dienen zur Stromeinspeisung und zum Abgriff der an dem Patienten abfallenden Spannung. Hierbei können dann in einem Schritt Strom und Spannung im selben Gerät gemessen werden, so daß die Impedanz (oder Admittanz) leicht berechnet werden kann.Bei einer vierpoligen Anwendung wird über zwei der Elektroden der Wechselstrom zugeführt, und über die beiden anderen Elektroden wird die abfallende Spannung gemessen. Stromquelle und Spannungsmeßgerät können somit räumlich getrennt sein. Damit aus der gemessenen Spannung die Impedanz berechnet werden kann, muß der von der Stromquelle abgegebene Strom (mit Betrag und Phase) bekannt sein. d. h. er muß entweder als Strom mit bekannter konstanter Amplitude (und Phase) erzeugt oder gemessen werden. Die vielfältigen Anwendungen der Impedanzmessung erfordern es, daß dies über einen großen Frequenzbereich gilt, etwa von 1 kHz bis 1 MHz, und dies bei unterschiedlicher Impedanz der Last.

An eine potentialfreie Wechselstromquelle werden daher hohe Anforderungen gestellt. Nicht nur müssen die oben genannten Sicherheitsvorschriften eingehalten werden, sondern die Quelle muß über einen großen Frequenzbereich einen konstanten Strom abgeben können.

Zur Potentialtrennung ist es bekannt, einen Transformator einzusetzen, der eine Patientenseite und eine davon potentialgetrennte Geräteseite definiert, wobei auf letzterer sämtliche netzbetriebenen elektrischen Bauteile, u. a. der Generator, angeordnet sind.

Bei jeder einfachen Transformatorkopplung ist insbesondere im Ausgang der Stromquelle der Frequenzbereich eingeschränkt und der Innenwiderstand der Quelle beschränkt (bedingt durch die Eigenschaften des realen Transformators), so dass das Erfordernis der Abgabe eines konstanten Stroms über einen großen Frequenzbereich nicht eingehalten werden kann.

Aus der US-Patentschrift 3 601 126 ist eine Wechselstromquelle für die größere Leistungen erfordernde Hochfrequenz-Chirurgie (Schneiden von Gewebeteilen, Koagulation von Blutgefäßen, Krebswachstumshemmung) mit einer Rückkoppplung des durch den Körper eines Patienten fließenden Laststroms bekannt. Sie besteht im wesentlichen aus einem Signal-Generator (RF/HF-Generator), einem Leistungsverstärker, einem Ausgangsübertrager, einem Strom-Sensor (Stromwandler-Transformator), einem Effektivwert-Detektor und einem Differenzverstärker (zum Istwert-Sollwert-Vergleich) mit nachfolgendem steuerbaren Verstärker, der den Leistungsverstärker steuert. Im Prinzip handelte es sich hier um eine isolierte, potenzialtrennende Wechselstromquelle, die einen geschlossenen Stromregelkreis mit einem Effektivwert-Detektor (Square-Law-Detector) beinhaltet.

Aus der US-Patentschrift 3 284 724 ist ein potentialfreier Oszillator mit einer Vorrichtung zur Stabilisierung der Amplitude des Oszillators bekannt, bei dem die Ausgangsamplitude des Oszillators konstant bleibt und über eine Konstantspannung einstellbar ist.

Es ist Aufgabe der Erfindung, eine potentialfreie Wechselstromquelle zum Erzeugen eines durch den Körper eines Patienten zu sendenden Messstroms zur Impedanz- oder Admittanzmessung bereitzustellen, die bei unterschiedlichen Lasten einen konstanten Strom abgibt und über größere Frequenzbereiche wie etwa 1 kHz bis 1 MHz einsetzbar ist.

Diese Aufgabe wird durch die kennzeichnenden Merkmale von Anspruch 1 oder Anspruch 2 gelöst.

Bei dieser Ausgestaltung entsteht in der zusätzlichen Wicklung aufgrund von Induktion eine Spannung, so daß bei der Rückkopplung auf diese Weise indirekt die Eigenschaften (Kernverluste, Streuinduktivitäten) des Transformators berücksichtigt werden.

Eine weitere Ausgestaltung der Lösung nach Anspruch 2 sieht vor, dass die zusätzliche Wicklung über einen verstellbaren Widerstand mit dem nicht invertierenden Eingang des Differenzverstärkers verbunden ist.

In den oben genannten Fällen wird also stets ein für den Ausgangsstrom der Stromquelle repräsentativer Spannungswert erhalten.

Vorteilhafte Ausgestaltungen der erfindungsgemäßen potentialfreien Wechselstromquelle werden anhand der folgenden Beschreibung unter Bezug auf die beigefügten Zeichnungen näher erläutert.

Es zeigen:
- Fig. 1: eine Ausführungsform der Wechselstromquelle und,
- Fig. 2: eine Ausgestaltung der Wechselstromquelle.

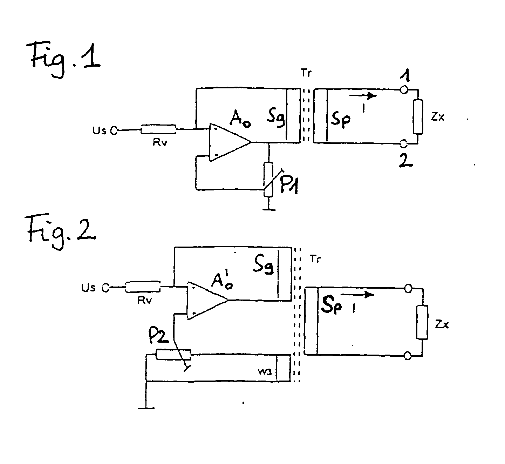

Bei der in Fig. 1 gezeigten Wechselstromquelle bewirkt ein Transformator Tr die von den gesetzlichen Sicherheitsvorschriften geforderte Potentialtrennung von einer (mit dem elektrischen Netz verbundenen) Geräteseite (im Bild links) und einer Patientenseite (im Bild rechts), wobei die patientenseitige Transformatorspule Sp zu zwei Anschlüssen 1 und 2 führt, die hier mit dem schematisch durch die Last Zx dargestellten Patienten verbindbar sind. Die Wechselstromquelle umfaßt ferner einen (in Fig. 1 nicht gezeigten) Generator, der ein Wechselspannungssignal Us erzeugt. Dieses Wechselspannungssignal wird über einen Widerstand Rv und einen Verstärker (bevorzugt Differenzverstärker) A₀ in einen Wechselstrom umgewandelt, der die geräteseitige Transformatorspule Sg erregt, so daß patientenseitig ein Wechselstrom erzeugt wird.

Das zu der geräteseitigen Spule Sg führende Ende des Widerstands Rv ist sowohl mit dem einen (in Fig. 1 oberen) Anschluß der geräteseitigen Spule Sg verbunden als auch mit dem invertierenden Eingang des Differenzverstärkers A₀. Der andere (in Fig. 1 untere) Anschluß der Spule ist mit dem Ausgang des Differenzverstärkers und gleichzeitig über ein Potentiometer P1 mit dem nicht invertierenden Eingang des Differenzverstärkers A₀ verbunden.

Bei der Wechselstromquelle aus Fig. 1 wird somit die an der geräteseitigen Spule Sg des Transformators Tr auftretende Spannung positiv rückgekoppelt. Dadurch wird der Innenwiderstand der Quelle erheblich erhöht, so dass in einem größeren Bereich von verschiedenen Lastwiderständen Zx eine konstante Stromamplitude gewährleistet ist. Über das Potentiometer P1 kann das Ausmaß der Rückkopplung eingestellt werden. Bei Bedarf kann die positive Rückkopplung frequenzabhängig gemacht werden, um die Wechselstromquelle für einen größeren Bereich einzurichten.

Eine weitere Verbesserung bringt die in Fig. 2 dargestellte Wechselstromquelle. Hier ist zusätzlich zur eigentlichen geräteseitigen Spule Sg geräteseitig eine weitere Spulenwicklung W3 vorgesehen, die allerdings nicht mit der eigentlichen Spule Sg verbunden, aber um denselben Kern gewickelt ist. Durch Induktion entsteht in dieser Wicklung (mit einer oder auch mehreren Windungen) eine induzierte Spannung. Statt dass, wie in der Wechselstromquelle in Fig. 1, direkt die an der geräteseitigen Spule Sg vorhandene Spannung zurückgekoppelt wird, wird hier dem nicht invertierenden Eingang des Differenzverstärkers A₀ die an der Wicklung W3 auftretende Spannung phasenrichtig zugeführt, wobei auch hier wieder ein Potentiometer P2 eingesetzt wird, über das das Maß der Rückkopplung eingestellt werden kann.

Die Wechselstromquelle aus Fig. 2 hat gegenüber der in Fig. 1 dargestellten Ausführungsform den Vorteil, daß nicht direkt die geräteseitige Spannung, die die Erzeugung des Stroms bewirkt, zurückgekoppelt wird, sondern dass hier ein Umweg über den Transformator erfolgt. Dadurch werden gewissermaßen dessen Eigenschaften bei der Rückkopplung mitberücksichtigt.

Die vorstehend genannten beiden Ausführungsformen beruhen auf einer Rückkopplung von Spannungen.

Die Erfindung stellt eine potentialfreie Wechselstromquelle bereit, die den gesetzlichen Sicherheitsvorschriften für die Impedanzmessung am menschlichen Körper genügt und gleichzeitig einen Wechselstrom abgibt, dessen Amplitude bei unterschiedlichen Lasten Zx und über einen großen Frequenzbereich, z. B. von 1 kHz bis 1 MHz, stabil ist.

## Patentansprüche

1. Potentialfreie Wechselstromquelle zum Erzeugen eines durch den Körper (Zx) eines Patienten zu sendenden Messstroms (I) zur Impedanz- oder Admittanzmessung,
mit einem Transformator (Tr), der eine Patientenseite und eine davon potentialgetrennte Geräteseite definiert und dessen patientenseitige Transformatorspule (Sp) zu Anschlüssen (1, 2) führt, die mit dem Patienten verbindbar sind, und
mit einem geräteseitigen Generator, der ein Wechselspannungssignal zur Steuerung einer Stromquelle erzeugt, die die geräteseitige Transformatorspule (Sg) erregt, so dass patientenseitig ein Wechselstrom (I) erzeugt wird, wenn die Anschlüsse (1, 2) über den Patienten als Last (Zx) miteinander verbunden sind,
**dadurch gekennzeichnet, dass** ein geräteseitiger Anschluss des Transformators (Tr) mit dem invertierenden Eingang eines Differenzverstärkers (A₀) und über einen Spannungsteiler (P1) mit dem nicht invertierenden Eingang und dem Ausgang des Differenzverstärkers (A₀) verbunden ist, so dass die an der geräteseitigen Wicklung des Transformators abfallende Wechselspannung positiv rückgekoppelt und mit zunehmendem Lastwiderstand angehoben wird.

2. Potentialfreie Wechselstromquelle zum Erzeugen eines durch den Körper (Zx) eines Patienten zu sendenden Messstroms (I) zur Impedanz- oder Admittanzmessung,
mit einem Transformator (Tr), der eine Patientenseite und eine davon potentialgetrennte Geräteseite definiert und dessen patientenseitige Transformatorwicklung (Sp) zu Anschlüssen (1, 2) führt, die mit dem Patienten verbindbar sind, und
mit einem geräteseitigen Generator, der ein Wechselspannungssignal zur Steuerung einer Stromquelle erzeugt, die die geräteseitige Transformatorwicklung (Sg) erregt, so dass patientenseitig ein Wechselstrom (I) erzeugt wird, wenn die Anschlüsse (1, 2) über den Patienten als Last (Zx) miteinander verbunden sind,
**dadurch gekennzeichnet, dass** ein geräteseitiger Anschluss des Transformators (Tr) mit dem invertierenden Eingang eines Differenzverstärkers (A₀') und ein anderer Anschluss mit dem Ausgang des Differenzverstärkers verbunden ist und dass geräteseitig zumindest eine zusätzliche Transformatorwicklung (W3) vorgesehen ist, die nicht mit dem Generator, aber mit dem nicht invertierenden Eingang des Differenzverstärkers (A₀') verbunden ist, so dass die an der geräteseitigen zusätzlichen Wicklung (W3) des Transformators (Tr) abfallende Wechselspannung positiv rückgekoppelt und mit zunehmendem Lastwiderstand angehoben wird.

3. Potentialfreie Wechselstromquelle nach Anspruch 2,
**dadurch gekennzeichnet, dass** die zusätzliche Wicklung (W3) über einen verstellbaren Widerstand (P2) mit dem nicht invertierenden Eingang des Differenzverstärkers (A₀') verbunden ist.

## Claims

1. Potential-free AC source for generating of a measurement current (I) to be transmitted to a patient for measuring impedance or admittance, comprising
a transformer (TR) having a patient's side and a potential-separated device side, the transformer winding (Sp) of the patient's side leading to terminals being connectable to the patient, and
a generator for generating an AC signal for controlling a current source energizing the transformer winding (Sg) on the device side so that an alternating current (I) is induced to the winding on the patient side if the terminals (1, 2) are connected with each other via the patient forming a load (Zx),
**characterized in that** the terminals of the transformer of the device side are connected to the inverting input terminal of a differential amplifier (A₀) and through a voltage divider (P1) to its non-inverting input terminal and to the output terminal of the differential amplifier so that the voltage that is dropped across the winding of the transformer on the device side is fed back positively and is increased when the load resistance increases.

2. Potential-free AC source for generating of a measurement current (I) to be transmitted to a patient for measuring impedance or admittance, comprising
a transformer (TR) having a patient's side and a potential-separated device side, the transformer winding (Sp) on the patient's side leading to terminals being connectable to the patient, and
a generator for generating an AC signal for controlling a current source energizing the transformer winding (Sg) on the device side so that an alternating current (I) is induced to the winding on the patient side if the terminals (1, 2) are connected with each other via the patient forming a load (Zx),
**characterized in that** one terminal of the device side of the transformer (Tr) is connected to the input terminal of a differential amplifier (A₀') and that the other terminal is connect to the output terminal of the differential amplifier and that at least one additional transformer winding (W3) is provided on the device side that is not connected with the generator but is connected to the non-inverting input terminal of the differential amplifier (A₀') so that the voltage dropped across the additional transformer winding (W3) on the device side of the transformer is fed back positively and increased when the load resistance increases.

3. Potential-free AC source according to claim 2, **characterized in that** the additional transformer winding (W3) is connected through an adjustable resistor (P2) with the non-inverting input terminal of the differential amplifier (A₀).

## Revendications

1. Source de courant alternatif à isolation galvanique, destinée à produire un courant de mesure (I) à envoyer à travers le corps (Zx) d'un patient pour une mesure d'impédance ou d'admittance,
comprenant un transformateur (Tr) qui définit un côté patient et un côté appareil isolé galvaniquement du précédent, et dont la bobine de transformateur (Sp), côté patient, mène à des bornes de connexion (1, 2) aptes à être reliées au patient, et
comprenant un générateur, côté appareil, qui produit un signal de tension alternative pour la commande d'une source de courant provoquant l'excitation de la bobine de transformateur (Sg), côté appareil, de sorte que du côté patient est produit un courant alternatif (I) lorsque les bornes de connexion (1, 2) sont reliées l'une à l'autre par l'intermédiaire du patient en tant que charge (Zx),
**caractérisée en ce qu'**une borne de connexion du transformateur (Tr), côté appareil, est reliée à l'entrée inverseuse d'un amplificateur différentiel (A₀) et, par l'intermédiaire d'un diviseur de tension (P1), à l'entrée non inverseuse et à la sortie de l'amplificateur différentiel (A₀), de sorte que la tension alternative chutant au niveau de l'enroulement du transformateur, côté appareil, est réinjectée positivement et augmentée avec une résistance de charge croissante.

2. Source de courant alternatif à isolation galvanique, destinée à produire un courant de mesure (I) à envoyer à travers le corps (Zx) d'un patient pour une mesure d'impédance ou d'admittance,
comprenant un transformateur (Tr) qui définit un côté patient et un côté appareil isolé galvaniquement du précédent, et dont l'enroulement de transformateur (Sp), côté patient, mène à des bornes de connexion (1, 2) qui peuvent être reliées au patient, et
comprenant un générateur, côté appareil, qui produit un signal de tension alternative pour la commande d'une source de courant provoquant l'excitation de la bobine de transformateur (Sg), côté appareil, de sorte que du côté patient est produit un courant alternatif (I) lorsque les bornes de connexion (1, 2) sont reliées l'une à l'autre par l'intermédiaire du patient en tant que charge (Zx),
**caractérisée en ce qu'**une borne de connexion, côté appareil, du transformateur (Tr), est reliée à l'entrée inverseuse d'un amplificateur différentiel (A₀') et une autre borne de connexion à la sortie de l'amplificateur différentiel, et **en ce que** du côté appareil, est prévu au moins un enroulement de transformateur supplémentaire (W3), qui n'est pas relié au générateur mais à l'entrée non inverseuse de l'amplificateur différentiel (A₀'), de sorte que la tension alternative chutant au niveau de l'enroulement supplémentaire (W3) du transformateur (Tr), côté appareil, est réinjectée positivement et augmentée avec une résistance de charge croissante.

3. Source de courant alternatif à isolation galvanique selon la revendication 2,
**caractérisée en ce que** l'enroulement supplémentaire (W3) est relié à l'entrée non inverseuse de l'amplificateur différentiel (A₀'), par l'intermédiaire d'une résistance réglable (P2).
